# EUROPÄISCHE PATENTSCHRIFT

(11) **EP 0 804 952 B1**
(45) Veröffentlichungstag und Bekanntmachung des Hinweises auf die Patenterteilung: **18.09.2002**
(21) Anmeldenummer: 97106252.6
(22) Anmeldetag: 16.04.1997
(51) Int. Cl.: B01D 9/00

(54) **Verfahren zur Herstellung von feinstteiligen Kristallisationsprodukten**
Process for the preparation of finely devided crystallisation products
Procédé de préparation de produits de cristallisation finement divisés

(30) Priorität: 29.04.1996 DE 19617085
(43) Veröffentlichungstag der Anmeldung: 05.11.1997
(73) Patentinhaber: BAYER AG, 51368 Leverkusen (DE)
(72) Erfinder: Schulte, Barbara, 29699 Bomlitz (DE); Hofmann, Stefan, Dr., 40764 Langenfeld (DE); Fellhölter, Andre, Dr., 51061 Köln (DE); Herold, Heiko, 41470 Neuss (DE); Behrendt, Frank, 51371 Leverkusen (DE)

(56) Entgegenhaltungen:
- WO-A-81/02688
- WO-A-94/07582
- DE-A- 4 118 185
- US-A- 4 985 239
- BORNSCHEIN M., MELEGARI P., BISMARCK C., KEIPERT S.: 'Mikro- und Nanopartikeln als Arzneistoffträgersysteme unter besonderer Berücksichtigung der Herstellungmethoden' DIE PHARMAZIE Bd. 44, Nr. 9, September 1989, Seiten 585 - 593

## Beschreibung

Die Erfindung betrifft ein Verfahren zur Herstellung von Kristallisationsprodukten mit einem mittleren Korndurchmesser < 1 µm durch Versprühen einer Lösung und gleichzeitige Verdampfung des Lösungsmittels. Dabei werden durch Verdüsung der Produktlösung und durch Verdampfung des Lösemittels Kristalle gebildet.

Die Erzeugung von Nanopartikeln ist nach vielfältigen Methoden möglich. Übersichten sind z.B. in [1] und in [2] aufgeführt.

Die dort beschriebenen Verfahren eignen sich aber fast ausschließlich zur Erzeugung von anorganischen Partikeln. Dies sind z.B. Fällverfahren in Tensidsystemen oder Mikroemulsionen zur Herstellung von Oxiden [3] oder Edelmetallen [4].

Einige Literaturstellen befassen sich auch mit Methoden zur Erzeugung von submikronen Partikeln aus organischen Stoffen, speziell pharmazeutischen Wirkstoffen.

In [5] ist die Präparation von schlecht wasserlöslichen Wirkstoffen aus Öl-in-Wasser Emulsionen beschrieben. Dabei wird die feinteilige Feststoffbildung aus den die gelöste Substanz enthaltenden Öl-Tropfen durch Abdampfung dieses Öls in einem Rotationsverdampfer erreicht. Die Konzentration des Feststoff (Cholesterylacetat) beträgt in etwa 1-2 Gew %, wenn Teilchen kleiner 1 Mikrometer erreicht wurden und der Gehalt an Tensid wird auf die organische Phase bezogen mit bis zu 20 Gew.-% angegeben. In eigenen Experimenten wurde festgestellt, daß diese Verfahrensweise nur für spezielle Stoffsysteme zum Erfolg führt, da mit zwei anderen Teststoffen (u.a. Naphthalin) keine stabilen kolloidalen Suspensionen erzeugt werden konnten.

Bioverfügbares Carotenoid-Hydrosol wird gemäß [6] durch Lösung des Carotenoids in einem wassermischbarem Lösemittel bei einer hohen Temperatur (> 100 °C) und anschließender schnellen wässrigen Fällung in Anwesenheit eines stabilisierenden Polymer-Kolloids erreicht. Die Größenordnung des Feststoffgehaltes und des Tensidgehaltes werden nicht genannt. Die Größenordnung der Teilchen liegt zwischen 50 nm und 500 nm.

Eine Übersicht über Mikro- und Nanopartikeln als Arzneistoffträgersysteme enthält die Literaturstelle [7]; allerdings sind die dort oberflächlich erwähnten Sprühverfahren nicht konkret für ein Beispiel der Nanopartikel-Herstellung nachvollziehbar.

Der Erfindung liegt die Aufgabe zu Grunde eine engverteilte bis monodisperse Kristallsuspension anorganischer und insbesondere organischer Verbindungen herzustellen, deren mittlerer Korngrößendurchmesser unterhalb von 1 Mikrometer liegt. Diese Korngröße muß zumindest über einen Zeitraum von mehreren Tagen bis 2 Wochen stabil vorliegen.

Die Belastung des zu kristallisierenden Stoffes durch hohe Temperaturen soll dabei vermieden werden. Der Feststoffgehalt der Suspension soll dabei auch auf Werte von größer als 1 Gew.-% eingestellt werden können.

Zur Lösung dieser Aufgabe dient ein Verfahren, bei dem eine Lösung versprüht und das Lösungsmittel verdampft wird. Dabei wird erfindungsgemäß die versprühte Lösung A in einer Gasatmossphäre auskristallisiert und gleichzeitig mit einem Tropfenschwarm einer tensidhaltigen Flüssigkeit B in Kontakt gebracht, wodurch eine Aerosolmischung A,B erzeugt wird, die anschließend als kolloide Kristallsuspension mit der tensidhaltigen Flüssigkeit als kontinuierliche Phase niedergeschlagen wird.

Vorzugsweise wird der Tropfenschwarm der tensidhaltigen Flüssigkeit B ebenfalls durch Versprühen erzeugt.

Das Verfahren wird entweder in der Weise ausgeübt, daß die Lösung A in den Tropfenschwarm der tensidhaltigen Flüssigkeit B oder vice versa die tensidhaltige Flüssigkeit B in die versprühte Lösung A eingedüst wird.

Vorteilhaft wird die Kristallsupension zumindest teilweise rezykliert und als tensidhaltige Flüssigkeit B versprüht.

Eine Variante, die vorteilhaft bei einem kontinuierlich durchgeführten Verfahren eingesetzt wird, besteht darin, daß die Kristallsuspension in einem Trennapparat aufkonzentriert wird und die abgereicherte Kristallsuspension rezykliert und als tensidhaltige Flüssigkeit B versprüht wird.

Um die Durchmischung des Tropfenschwarms mit der versprühten Lösung A zu intensivieren, kann die gebildete Aerosolmischung A,B durch einen Gaswäscher geleitet werden.

Als Lösungsmittel für die zu kristallisierende Verbindung werden vorzugsweise leichtflüchtige Flüssigkeiten eingesetzt, die bei Zimmertemperatur einen Dampfdruck > 0,1 mbar aufweisen.

Als tensidhaltige Flüssigkeit wird zweckmäßig Wasser mit einem Tensid-Zusatz verwendet.

Eine weitere Option besteht darin, daß der Lösung A ebenfalls ein Tensid zugesetzt wird.

Eine wichtige Anwendung des Verfahrens besteht darin, daß die Kristallsuspension als Formulierung für parenterale Arzneistoffzubereitungen verwendet wird.

Die Herstellung von Partikeln im Größenbereich kleiner 1 Mikrometer ist von großem Interesse für industrielle Anwendungen, da das Eigenschaftenprofil der kolloidalen Partikeln gegenüber dem der konventionellen Partikeln oberhalb der 1 Mikrometergrenze deutlich unterschiedlich ist. Für organische Partikel, speziell pharmazeutische Wirkstoffe, sind dies z.B. das veränderte Lösevermögen ("Bioverfügbarkeit") oder die Verwendbarkeit als parenterale Lösung für pharmazeutische Zwecke trotz des partikulären Charakters ("Depoteffekt").

Die Erzeugung von Nanopartikeln durch direkte Herstellung nativer Kristalle hat deutlichen Vorteil gegenüber der Herstellung durch mechanisches Zerkleinern (z.B. Perlmahlen). Bei der Zerkleinerung entstehen Bruchflächen, die energetisch betrachtet eine verstärkte Tendenz zum Ausheilen und Wachsen aufweisen, wodurch die Korngröße der Partikel durch Rekristallisation oder Agglomeration wieder ansteigt.

Das vorgestellte Verfahren eignet sich insbesondere für die Erzeugung von organischen Kristallen, wohingegen die meisten andere Verfahren zur Nanopartikelerzeugung durch die Wahl der Prozeßparameter (z.B. hohe Temperaturen) nur für die Herstellung von anorganischen Feinstpartikeln einsetzbar sind.

Die beschriebene Methode der tensidstabilisierten Sprühkristallisation hat auch den Vorteil, daß keine Voremulsion der zu kristallisierenden Substanz hergestellt werden muß.

Die statt dessen verwendete Lösung des gewünschten Feststoffes kann dagegen im Rahmen der Löslichkeit flexibel in der Konzentration und bei geeigneter Wahl des Lösemittels auch sehr hoch in dem Gehalt an Produkt eingestellt werden.

Die zur Erzeugung von Emulsionen notwendige Dispergierungs- und Homogenisierungs-Energie (z.B. durch Ultra-Turax oder Hochdruckhomogenisierung) wird eingespart. Ebenso ist die unter Umständen kritische Suche einer Tensidkombinationen zur Stabilisierung des zu emulgierenden Stoffsystems nicht notwendig.

Zur Verarbeitung der Lösung kann ein einfaches, nicht pulsierendes Pumpensystem (z.B. eine Spritzenpumpe) verwendet werden. Es sind keine hohen Drücke (> 10 bar) oder hohen Temperaturen (> 100°C) notwendig. Dies macht die Methode besonders für temperaturempfindliche organische Stoffe anwendbar.

Durch die Verdüsung der Lösung in feine Tropfen wird der Dampfdruck des Lösemittels heraufgesetzt, so daß auch Umgebungsdruck oder leichtes Vakuum zur Entfernung des Lösemittels ausreicht. Dies ist ein Vorteil im Gegensatz zu Methoden, bei denen das Lösemittel aus der kompletten Emulsion im hohen Vakuum abgezogen werden muß.

Die Partikel bilden sich aus den verdampfenden Tröpfchen der verdüsten Lösung und werden in der erfindungsgemäßen Prozeßführung noch im Verlauf ihrer Trocknung von tensidhaltigen Wassertropfen abgefangen. Die geeignet ausgewählten handelsüblichen Tenside (z.B. im Hinblick auf ein hohes Benetzungs- und Stabilisierungs-Vermögen an Grenzflächen) umhüllen den erzeugten Kristallit und verhindern so weitestgehend weitere Stoffaustauschvorgänge. Daher können Agglomerationsvorgänge und Rekristallisationsvorgänge der kolloidalen Teilchen verhindert werden. Die verwendete Menge an tensidhaltigem Wasser, das im Kreislauf verwendet wird (Rezyklierung), kann flexibel auf die gewünschte Feststoffkonzentration in der Produktsuspension eingestellt werden. Eine in den Kreislauf geschaltete Vorfiltration kann gegebenenfalls einen aufkonzentrierten Teilstrom mit den erzeugten Nanopartikeln abziehen.

Die Prozeßführung eignet sich sowohl für einen kontinuierlichen als auch einen batchweisen Betrieb.

Im folgenden wird die Erfindung an Hand von Ausführungsbeispielen und Zeichnungen näher beschrieben. Es zeigen:
Fig. 1 das Prinzip einer Versuchsanlage
Fig. 2 das Konzept einer technischen Anlage und
Fig. 3 eine elektronenmikroskopische Aufnahme der gebildeten Kristallite

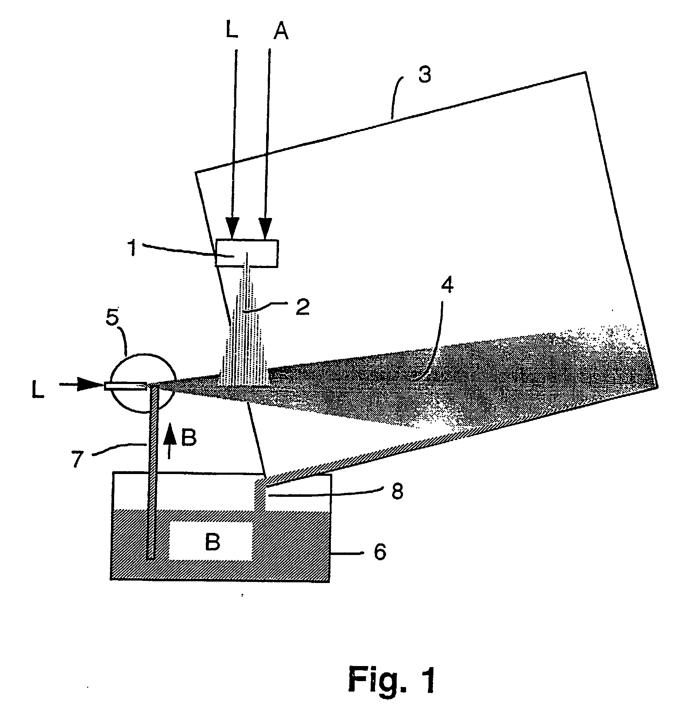

Der interessierende zu kristallisierende Stoff A wird in einem leichtflüchtigen Lösemittel gelöst. Diese Lösung A wird gemäß Fig. 1 durch den 0.4 mm-Kanal einer Zweistoffdüse 1 gefördert, während die zum Betrieb der Zweistoffdüse erforderliche Druckluft L mit einem absoluten Druck von z.B. 3 bar über 6 Bohrungen zum engsten Querschnitt des konzentrischen Spaltes der Zweistoffdüse mit einem äquivalenten Durchmesser von 0.55 mm strömt. Dadurch werden feinste Tröpfchen (Aerosol) in Form eines Sprühkegels 2 in der Kammer 3 erzeugt. Während das Lösungsmittel verdampft, kristallisiert der gelöste Stoff feinstteilig aus. Der Sprühkegel 2 wird, bevor er auf die Wandflächen der umgebenden Sprühkammer 3 auftreffen kann, von einem in die Kammer 3 eingedüsten Tropfenschwarm 4 durchdrungen, der aus einer stabilisierenden Phase besteht. Als stabilisierende Phase wird z.B. Wasser mit einem Zusatz von 10 Gew.-% Tensid eingesetzt. Der Tropfenschwarm 4 wird mit Hilfe einer Zerstäubungsvorrichtung 5 erzeugt. Auf diese Weise wird eine Benetzung der frisch erzeugten Nano-Kristallpartikel mit Tensiden erreicht. Zur Zerstäubung des tensidhaltigen Wassers, das in einem Reservoir 6 vorgelegt wird, kann gem. Figur 1 ein einfacher Glaszerstäuber verwendet werden, der wie auch die Zweistoffdüse 1 mit Druckluft L zur Versprühung des über das Steigrohr 7 zugeführten Tensid-Wasser-Gemisches beschickt wird.

Die in der Kammer 3 auf Grund der Durchdringung des Sprühkegels 2 mit dem Tropfenschwarm 4 erzeugte Aerosolmischung A,B wird an den Wänden der Kammer 3 niedergeschlagen und bildet die gewünschte kolloidale Kristallsuspension, die aus der Kammer 3 freifließend als Flüssigkeitsschicht 8 in das Reservoir 6 abfließt. Zu Beginn des Verfahrens war in dem Behälter 6 nur das tensidhaltige Wasser vorgelegt. Da während des Verfahrens durch die Zweistoffdüse 1 ständig neue Lösung zugeführt wird, reichert sich die Feststoffkonzentration im Behälter 6 immer weiter an. Das Verfahren wird abgebrochen, wenn die Kristallsuspension im Behälter 6 die gewünschte Endkonzentration erreicht hat.

Ein wichtiger Schritt bei der Kristallisation ist der Lösemittelentzug. Daher wird eine möglichst spontane Verdampfung aus der produkthaltigen Lösung A angestrebt.

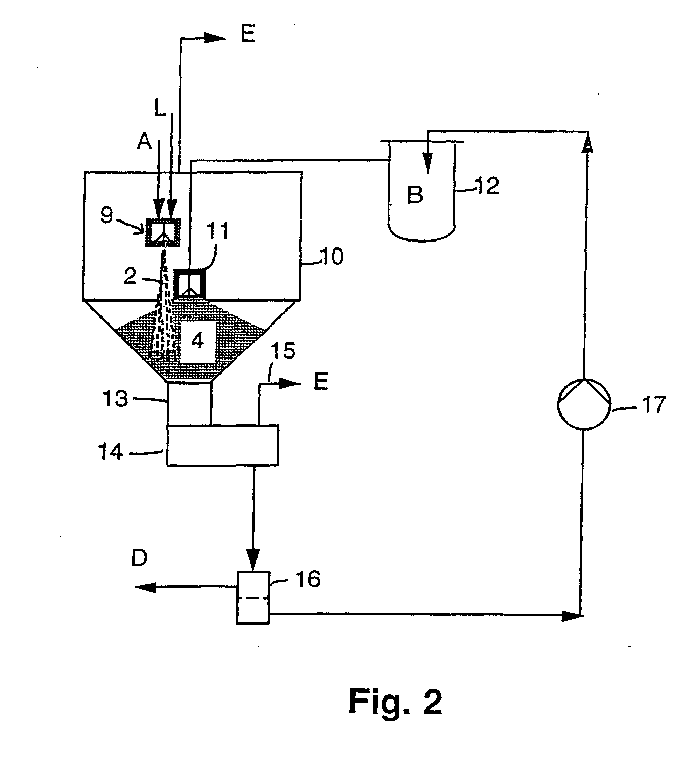

Ein technisches Konzept für die Durchführung des erfindungsgemäßen Verfahrens ist in Fig. 2 dargestellt.

Die produkthaltige Flüssigkeit A wird mit Druckluft L durch eine Miniatur-Zweistoffdüse 9 in einer geschlossenen Kammer 10 zu einem Sprühkegel 2 zerstäubt. Dabei bilden sich, wie schon an Hand von Fig.1 beschrieben, auf Grund der spontan einsetzenden Verdampfung des Lösungsmittels feinste Kristallite. Bevor dieser Sprühkegel auf die Wandflächen der umgebenden Kammer 10 auftreffen kann, durchtritt er einen die Kristallite benetzenden Tropfenschwarm 4 aus tensidhaltigen Wassertropfen, der mit Hilfe einer handelsüblichen Einstoff- oder Zweistoffdüse 11 erzeugt wird. Die Minidüse 9 wird mit tensidhaltigen Wasser B beschickt, das in einem Behälter 12 vorgelegt ist . An den Ausgang der Kammer 10 schließt sich ein Venturi-Wäscher 13 an, in dem die tensidhaltigen Wassertropfen in intensiven Kontakt mit dem aus der Miniatur-Zweistoffdüse 9 austretenden Produkt/Luft-Aerosol-Strahl (Sprühkegel 2) gebracht werden. Das resultierende, die Kristalle enthaltende Tropfen-Aerosol strömt von dort in einen Gasabscheider 14. Die entstehende Abluft E aus dem zur Versprühung verwendeten Luftstrom L und den verdampfenden Lösemittelanteilen aus der Lösung A wird zum Teil aus der Sprühkammer 10 direkt abgeleitet und zum anderen Teil bei der Sammlung des Aerosols im Abscheider 14 durch den Stutzen 15 abgeleitet und einer Wiederaufarbeitung bzw. Entsorgung zugeführt. Zur besseren Entfernung von Lösungsmitteldämpfen kann die Kammer 10 auch mit leichtem Unterdruck betrieben werden.

Die Kristallsuspension im Abscheider 14 enthält die Nanometer-großen Produktkristalle, die in einem weiteren Schritt durch ein Querstromfilter 16 angereichert und in Form einer Suspension D mit höherem Feststoffgehalt ausgeschleust werden, während das tensidhaltige Filtrat mittels der Pumpe 17 in den Behälter 12 rezykliert wird.

### Versuchsbeispiel

In der Apparatur gemäß Figur 1 wurde das erfindungsgemäße Verfahren im Labormaßstab mit folgenden Daten durchgeführt:
a) Die Temperatur betrug ca 20 °C (Raumtemperatur).
b) Die Lösung A bestand aus einer 5 Gew.-% Naphthalin-Chloroform Lösung mit einer Dichte von 1,5 g/ml (0,75 g Naphthalin in Chloroform, insgesamt 15 g). Chloroform hat bei Raumtemperatur einen Dampfdruck von 0.211 mbar und eine Wasserlöslichkeit von 0.82 Gew.-%.
c) Die tensidhaltige Flüssigkeit B bestand aus 3,17 g ®Triton X 100 (nichtionisches Tensid), 1,93 g ®Marlon A 375 (anionisch: Nadodecylbenzolsulfonat) und 44,9 g Wasser (Tensidanteil etwa 10 Gew.-%). Diese Tensid-Mischung besitzt ein hohes Benetzungs- und Stabilisierungs-Vermögen an Grenzflächen. Bei den genannten Tensiden handelt es sich um preisgünstige technische Handelsprodukte.
d) Zur dosierten Zuführung der Lösung A wurde eine Spritzenpumpe verwendet. Der Volumenstrom der Lösung A betrug während der 15-min Versuchsdauer 60 ml/h.
e) Die Druckluft L wurde mit einem absoluten Druck von 3 bar zugeführt, was in der Zweistoffdüse 1 einem Durchsatz von etwa 500 l/h Luft entspricht.

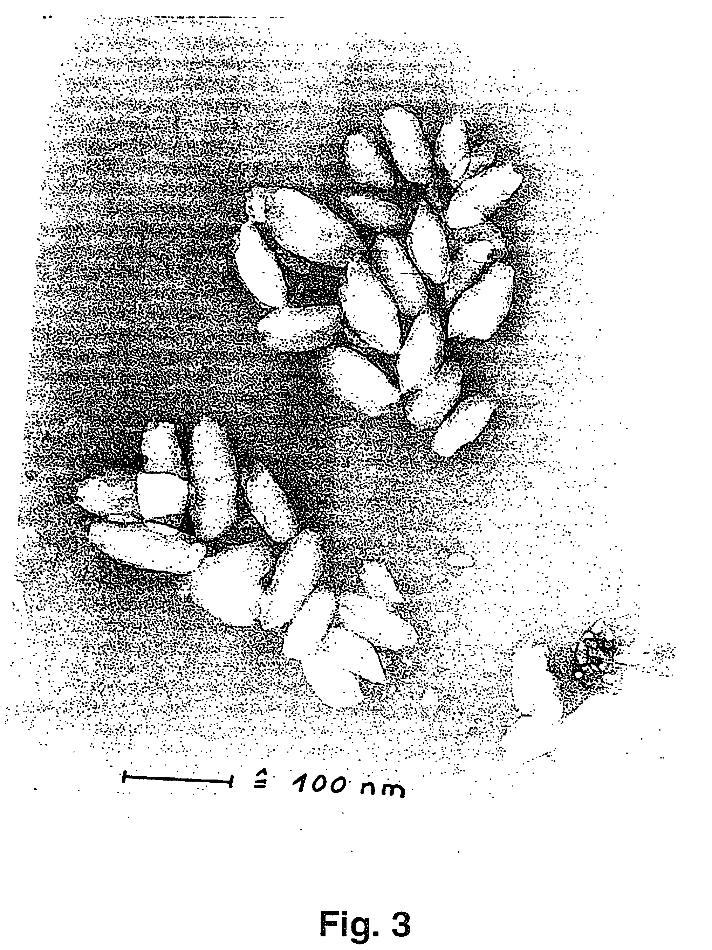

Proben der so hergestellten Kristallsuspension wurden durch Elektronenmikroskopie (TEM-Aufnahme, siehe Figur 3) auf die erreichte Korngrößenverteilung hin untersucht. In der Aufnahme sind die annähernd ellipsoidförmigen Kristallite gut zu erkennen. 8 mm auf dem Bild entsprechen 1 Mikrometer der Probe. Die Partikel liegen im Größenbereich von 100 nm mit einer engen Korngrößenverteilung. Auch nach einer Standzeit von 19 Tagen konnten noch keine Rekristallisate beobachtet werden. Rechnerisch (ohne Berücksichtigung der Verluste) ergab sich eine Feststoffkonzentration von 1,5 Gew.-% und eine Tensidkonzentration von 10 Gew.-% in der kolloidalen Kristallsuspension.
[1] Preparation of monodispersed colloidal particles Sugimoto, T. Advances in colloid and interface science 28 (1987) S.65-108
[2] The world of fine particles (Feinste Partikel, hergestellt durch Ausfälle oder chemische Reaktionen mit Tropfen) Matijevic, E. Chemtech 21 (1991) 3, S.176-181
[3] Verfahren zur Herstellung nanoskaliger Oxidteilchen Schmidt, H. DE 4 118 185 A1 (3.6.1991)
[4] Suspension of platinum group metal particles in microemulsion with uniform particle size, useful for making catalysts Stenius, P. WO 81/ 02688 (1.10.1981)
[5] A method for the preparation of submicron particles of sparingly watersoluble drugs by precipitation in oil-in-water emulsions: part I & part II Sjöström Brita, Kronberg B., Carlfors J. J. Pharm. Sci. 82 (6), (1993), S. 579-589
[6] Preparation and characterization of microdisperse bioavailable carotenoid Hydrosols D. Horn Die angewandte makromolekulare Chemie 166/167 (1989) S.139-153 (2874)
[7] Mikro- und Nanopartikeln als Arzneistoffträgersysteme unter besonderer Berücksichtigung der Herstellungsmethoden Bornschein M., Melegari P., Bismarck Ch., Keipert,S. Die Pharmazie 44(9) (1989) S.585-593

## Patentansprüche

1. Verfahren zur Herstellung von Kristallisationsprodukten mit einem mittleren Korndurchmesser < 1 µm durch Versprühen einer Lösung und gleichzeitige Verdampfung des Lösungsmittels, **dadurch gekennzeichnet, daß** die versprühte Lösung A in einer Gasatmossphäre auskristallisiert und gleichzeitig mit einem Tropfenschwarm (4) einer tensidhaltigen Flüssigkeit B in Kontakt gebracht wird, wodurch eine Aerosolmischung A,B erzeugt wird, die anschließend als kolloide Kristallsuspension mit der tensidhaltigen Flüssigkeit B als kontinuierliche Phase niedergeschlagen wird.

2. Verfahren nach Anspruch 1, **dadurch gekennzeichnet, daß** der Tropfenschwarm (4) der tensidhaltigen Flüssigkeit ebenfalls durch Versprühen erzeugt wird.

3. Verfahren nach Anspruch 2, **dadurch gekennzeichnet, daß** die Lösung A in den Tropfenschwarm (4) der tensidhaltigen Flüssigkeit B oder vice versa die tensidhaltige Flüssigkeit B in die versprühte Lösung A eingedüst wird.

4. Verfahren nach Anspruch 1 bis 3, **dadurch gekennzeichnet, daß** die Kristallsupension zumindest teilweise rezykliert und als tensidhaltige Flüssigkeit B versprüht wird.

5. Verfahren nach Anspruch 1 bis 3, **dadurch gekennzeichnet, daß** die Kristallsuspension in einem Trennapparat (16) aufkonzentriert wird und die abgereicherte Kristallsuspension rezykliert und als tensidhaltige Flüssigkeit B versprüht wird.

6. Verfahren nach Anspruch 1 bis 5, **dadurch gekennzeichnet, daß** die Aerosolmischung A,B zur Intensivierung der Durchmischung durch einen Gaswäscher (13) geleitet wird.

7. Verfahren nach Anspruch 1 bis 6, **dadurch gekennzeichnet, daß** das Lösungsmittel für die zu kristallisierende Verbindung bei Zimmertemperatur einen Dampfdruck > 0,1 mbar aufweist.

8. Verfahren nach Anspruch 1 bis 7, **dadurch gekennzeichnet, daß** als tensidhaltige Flüssigkeit B Wasser mit einem Tensid-Zusatz verwendet wird.

9. Verfahren nach Anspruch 1 bis 8, **dadurch gekennzeichnet, daß** der Lösung A ebenfalls ein Tensid zugesetzt wird.

10. Verfahren nach Anspruch 1 bis 9, **dadurch gekennzeichnet, daß** die Kristallsuspension als Formulierung für parenterale Arzneistoffzubereitungen verwendet wird.

## Claims

1. Process for producing crystallization products having an average particle diameter of < 1 µm by atomizing a solution and simultaneously evaporating the solvent, **characterized in that** the atomized solution A is crystallized out in a gas atmosphere and simultaneously contacted with a cloud (4) of droplets of a surfactant-containing liquid B to form an aerosol mixture A, B which is subsequently deposited as a colloidal crystal suspension having the surfactant-containing liquid B as a continuous phase.

2. Process according to Claim 1, **characterized in that** the cloud of drops (4) of the surfactant-containing liquid is also produced by atomization.

3. Process according to Claim 2, **characterized in that** solution A is injected into the cloud of drops (4) of the surfactant-containing liquid B or, vice versa, the surfactant-containing liquid B is injected into the atomized solution A.

4. Process according to Claims 1 to 3, **characterized in that** the crystal suspension is at least partially recycled and atomized in the form of a surfactant-containing liquid B.

5. Process according to Claims 1 to 3, **characterized in that** the crystal suspension is concentrated in a separating apparatus (16) and the depleted crystal suspension is recycled and atomized in the form of a surfacant-containing liquid B.

6. Process according to Claims 1 to 5, **characterized in that** the aerosol mixture A, B is passed through a gas scrubber (13) in order to intensify the mixing process.

7. Process according to Claims 1 to 6, **characterized in that** the solvent for the compound to be crystallized has a vapour pressure of >0.1 mbar at room temperature.

8. Process according to Claims 1 to 7, **characterized in that** the surfactant-containing liquid B is water to which a surfactant has been added.

9. Process according to Claims 1 to 8, **characterized in that** solution A likewise has a surfactant added to it.

10. Process according to Claims 1 to 9, **characterized in that** the crystal suspension is used as a formulation for parenteral drug preparations.

## Revendications

1. Procédé de préparation de produits de cristallisation d'un diamètre granulaire moyen < 1 µm par pulvérisation d'une solution et évaporation simultanée du solvant, **caractérisé en ce que** la solution A pulvérisée précipite en cristaux dans une atmosphère gazeuse et est simultanément mise en contact avec une nuée de gouttes (4) d'un liquide B contenant un tensioactif, ce qui produit un mélange aérosol A,B qui précipite ensuite en phase continue avec le liquide B contenant un tensioactif sous forme de suspension cristalline colloïde.

2. Procédé selon la revendication 1, **caractérisé en ce que** la nuée de gouttes (4) du liquide contenant un tensioactif est également produite par pulvérisation.

3. Procédé selon la revendication 2, **caractérisé en ce que** la solution A dans la nuée de gouttes (4) du liquide B contenant un tensioactif ou inversement le liquide B contenant un tensioactif dans la solution A pulvérisée est injecté.

4. Procédé selon la revendication 1 à 3, **caractérisé en ce que** la suspension cristalline est au moins partiellement recyclée et est pulvérisée comme liquide B contenant un tensioactif.

5. Procédé selon la revendication 1 à 3, **caractérisé en ce que** la suspension cristalline est concentrée dans un appareil de séparation (16) et que la suspension cristalline appauvrie est recyclée et pulvérisée comme liquide B contenant un tensioactif.

6. Procédé selon la revendication 1 à 5, **caractérisé en ce que** le mélange aérosol A,B est conduit à travers un laveur de gaz (13) pour intensifier le mélange.

7. Procédé selon la revendication 1 à 6, **caractérisé en ce que** le solvant présente à température ambiante pour le composé à cristalliser une pression de vapeur > 0,1 mbar.

8. Procédé selon la revendication 1 à 7, **caractérisé en ce qu'**on utilise comme liquide B contenant un tensioactif de l'eau avec une addition de tensioactif.

9. Procédé selon la revendication 1 à 8, **caractérisé en ce qu'**un tensioactif est également ajouté à la solution A.

10. Procédé selon la revendication 1 à 9, **caractérisé en ce que** la suspension cristalline est utilisée comme formulation pour des préparations médicinales parentérales.
